# EUROPEAN PATENT APPLICATION

(11) **EP 4 603 077 A1**
(43) Date of publication of application: **20.08.2025**
(21) Application number: 23876491.4
(22) Date of filing: 20.09.2023
(51) Int. Cl.: A61K 8/43, A61K 8/42, A61K 8/40, A61Q 1/14, A61Q 5/02, A61Q 19/10

(54) **GEL COMPOSITION, AND PREPARATION AND USE THEREOF**

(30) Priority: 13.10.2022 CN 202211255910; 27.12.2022 CN 202211686135
(71) Applicant: Suzhou Oulit Biopharm Co., Ltd., Jiangsu 215411 (CN)
(72) Inventor: ZHANG, Jian, Suzhou, Jiangsu 215122 (CN)
(74) Representative: Winger
(86) International application number: PCT/CN2023/120158
(87) International publication number: WO 2024/078286

(57) **Abstract**

Disclosed in the present invention are a gel composition, a related gel, and a preparation method therefor and the use thereof. The gel composition is based on a long-chain N-acyl amino acid, contains no thickener, is transparent/semitransparent in appearance, is mild and non-irritant, and has very good heat resistance and cold resistance. A related gel product is convenient to carry and applicable to various environments and various usage scenarios, and is very easy to rinse off after use. The gel composition provided in the present invention can be applied to a cleaning composition, a washing composition, a cosmetic composition, a skin care composition, a toothpaste composition, etc.

## Description

### TECHNICAL FIELD

The present disclosure relates to the technical field of gel products, and in particular, to a gel composition comprising a N-long-chain acyl amino acid and a basic amino acid/inorganic base, and related preparation and use in fields of cosmetics, detergents, toothpaste, etc.

### BACKGROUND

Gel products feature certain aesthetic properties, the absence of greasy feelings, ease to apply and clean, etc., and are widely used in fields of cosmetics, detergents, toothpaste, and the like due to their clear and transparent/semitransparent appearance, unique rheological properties, and small suspended particles.

At present, gels are formed mainly by adding various polymeric thickening agents or gelling agents. Commonly used thickening agents include cellulose, mucopolysaccharides, polyvinyl alcohol, gums, sodium polyacrylate, polyvinyl pyrrolidone, vinyl polymers, and the like. Specifically, those commonly used include starch, gelatin, sodium alginate, guar gum, chitin gum, gum arabic, xanthan gum, soy protein gum, polyacrylamide, polyvinyl alcohol, polyvinylpyrrolidone, modified paraffin resin, carbomer resin, styrene butadiene rubber, and the like. For the AES system, sodium chloride may also be used for thickening.

CN107875037A discloses a skin softening gel containing a colored wax, which requires the use of acrylic acid (acrylate)/C10-30 alkanol acrylate cross-linked polymer. However, arginine is present in the formulation in an amount of 0.2-0.3% only and is thus not used for forming a gel.

CN106361596A discloses an amino acid foaming facial cleansing gel, which requires the use of acrylate cross-linked polymer, hydroxyethyl cellulose, and the like in the formulation. In a surfactant system, hydroxypropyl methyl cellulose delays the loss of water through gelling.

JP2021161104A discloses a gel hair cosmetic composition, containing polyvinylpyrrolidone as component (A), diglycerin as component (B), arginine as component (C), and xanthan gum as component (D), wherein arginine is added in an amount of 0.01 wt% or greater but less than 0.3 wt% for the purpose of imparting a soft setting force to the hair after application and suppressing flaking.

CN104812373A discloses an oral gel for sensitivity and dental pain. The matrix of the gel contains an anionic polymer and a basic amino acid, and the anionic polymer contains cross-linked poly(acrylic acid).

US4010254A discloses a gel based on a vinyl ether-maleic anhydride copolymer neutralized with a basic amino acid. The aqueous solution of vinyl ether-maleic anhydride copolymer has gel-forming properties but features an pH acidic value and irritant nature. The basic amino acid is added primarily for neutralization and reducing irritation, rather than contributing to gel formation.

JP2004123575A discloses a topical gel composition, in which a water-soluble vinyl polymer is used as the matrix for the aqueous gel agent, and a basic amino acid (such as arginine) is added for moisturizing.

The above documents adopt conventional gel forming techniques, in which polymeric thickening agents are used to prepare the gel, and basic amino acids are present in small amounts and do not serve for gelling. Conventional gel systems are challenged by various issues. For example, in a system where a salt is present, it may sometimes be difficult to gel a product with a polymeric thickening agent, and a large amount of a water-soluble polymeric thickening agent may be added, which adversely affects the functionality of the product and the user experience such as skin feel. For another example, the addition of thickening agents/gelling agents may sometimes lead to discoloring at a high temperature, suppress the foaming properties of the surfactant, and affect the skin feel due to the macromolecules during cleansing, leading to pseudo slipperiness, incomplete rinsing, a strong sense of residue, and the like.

CN104105686A discloses a gelling agent, comprising a basic amino acid derivative having the following structure, which exerts its gelling ability in an aqueous system and can be used in various cosmetics.

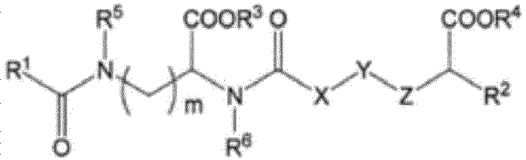

CN104114535B discloses a zwitterionic basic amino acid derivative having the following structure, which exerts its gelling ability in an aqueous system as well.

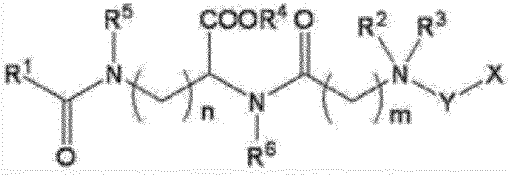

In the above documents, amino acid derivatives are used as gelling agents. However, the dependence on the specific structures of the amino acid derivatives and cost-inefficiency make the derivatives unsuitable for mass production and application.

Amino acid surfactants feature mildness, low toxicity, good biodegradability, good wettability, foamability, antistatic properties, compatibility, antibacterial properties, and the like, and are thus widely used in daily chemical formulations such as skin care products, body wash gels, facial cleansers, detergents, and toothpaste, as well as industries such as biopharmaceuticals and the like. The use of amino acid surfactants in the formulation faces even greater challenges in preparing gel products.

Several studies have been initiated on gel products comprising amino acid surfactants. For example, EP1505954A1 discloses a potent foaming cleansing gel containing an acyl amino acid surfactant, the cosmetic cleansing gel comprising: a) water at a concentration of 60 to 90 wt%, b) a thickening agent at a concentration of 0.1 to 5 wt%, and c) an acyl amino acid surfactant at a concentration of 0.1 to 6 wt%. For gelling, the gel uses polyacrylate and/or xanthan gum and/or acrylate/alkyl acrylate crosspolymers as the thickening agent.

CN1798821A discloses a gel composition comprising a N-acyl amino acid (A) and at least 1 oily matrix (B), wherein the N-acyl amino acid is present in a small amount (about 5%) for regulating the touch of oily matrix.

Despite the studies, the thickening of amino acid surfactant systems is still challenging. Manufacturers have to provide related products with a foaming pump or use amino acid surfactants as the cosurfactant of conventional petroleum-based surfactants such as fatty alcohol polyoxyethylene ether sulfate (AES), sodium dodecyl sulfate, sodium dodecylbenzenesulfonate, and the like, rather than the primary surfactant.

The development of high-quality, multifunctional, environment-friendly surfactants has become a main orientation of the surfactant industry, and to replace conventional petroleum-based surfactants with amino acid surfactants is the progress trend of the industry. Unilever has announced the halving of petroleum-based product use in 2025 and phaseout by 2030. Therefore, it is necessary to solve the problem of preparing a gel product when an amino acid surfactant is used as the primary surfactant, or specifically, preparing a gel product without the use of conventional thickening agents/gelling agents.

### SUMMARY

In view of the above, in one aspect, the present disclosure is intended to provide a gel composition, comprising an amino acid surfactant as the primary surfactant.

In another aspect, the present disclosure provides a gel that is transparent/semitransparent in appearance, mild, and non-irritant, and features excellent thermo- and cryo-resistance. As a product, the gel features good portability for various non-domestic circumstances such as outdoors, and ease to rinse in use.

In still another aspect, the present disclosure is intended to provide a preparation method with a simple process, cost-efficiency, and ease of industrial production, to readily acquire gel products based on amino acid surfactants.

In a last aspect, the present disclosure provides a method for imparting gel characteristics to a cleansing composition.

Specifically, the present disclosure discloses the following schemes.
[1] A gel composition, comprising: (a) a N-long-chain acyl amino acid, (b) a basic amino acid, and (c) water, wherein the gel is formed by self-thickening of the N-long-chain acyl amino acid and the basic amino acid;
   or, comprising: (a) a N-long-chain acyl amino acid, (b) a basic amino acid, (c) water, and (d) a N-long-chain acyl amino acid dipeptide, wherein the gel is formed by self-thickening of the N-long-chain acyl amino acid, the basic amino acid, and the N-long-chain acyl amino acid dipeptide;
   or, comprising: (a) a N-long-chain acyl amino acid, (e) an inorganic base, and (c) water, wherein the gel is formed by self-thickening of the N-long-chain acyl amino acid and the inorganic base;
   or, comprising: (a) a N-long-chain acyl amino acid, (e) an inorganic base, (c) water, and (d) a N-long-chain acyl amino acid dipeptide, wherein the gel is formed by self-thickening of the N-long-chain acyl amino acid, the inorganic base, and the N-long-chain acyl amino acid dipeptide.
[2] A gel composition, comprising: (a) a N-long-chain acyl amino acid, (b) a basic amino acid, and (c) water, wherein the percentage by weight of the N-long-chain acyl amino acid is 30 wt% or greater, preferably 33 wt% or greater, and more preferably 35 wt% or greater, and the percentage by weight of the basic amino acid is 15 wt% or greater, preferably 17 wt% or greater, and more preferably 20 wt% or greater;
   or, comprising: (a) a N-long-chain acyl amino acid, (b) a basic amino acid, (c) water, and (d) a N-long-chain acyl amino acid dipeptide, wherein the sum of the percentages by weight of the N-long-chain acyl amino acid and N-long-chain acyl amino acid dipeptide is 20 wt% or greater, preferably 23 wt% or greater, and more preferably 25 wt% or greater, and the percentage by weight of the basic amino acid is 10 wt% or greater, preferably 12 wt% or greater, and more preferably 14 wt% or greater;
   or, comprising: (a) a N-long-chain acyl amino acid, (b) a basic amino acid, and (c) water, wherein the percentage by weight of the N-long-chain acyl amino acid is 20 wt% or greater, preferably 23 wt% or greater, and more preferably 25 wt% or greater, and the molar ratio of the N-long-chain acyl amino acid to the basic amino acid is 1:0.98 to 1:0.85, preferably 1:0.96 to 1:0.88, and more preferably 1:0.95 to 1:0.90.
[3] A gel composition, comprising: (a) a N-long-chain acyl amino acid, (e) an inorganic base, and (c) water, wherein the percentage by weight of the N-long-chain acyl amino acid is 40 wt% or greater, preferably 43 wt% or greater, and more preferably 45 wt% or greater, and the percentage by weight of the inorganic base is 5.5 wt% or greater, preferably 6 wt% or greater, and more preferably 6.5 wt% or greater;
   or, comprising: (a) a N-long-chain acyl amino acid, (e) an inorganic base, (c) water, and (d) a N-long-chain acyl amino acid dipeptide, wherein the sum of the percentages by weight of the N-long-chain acyl amino acid and N-long-chain acyl amino acid dipeptide is 30 wt% or greater, preferably 33 wt% or greater, and more preferably 35 wt% or greater, and the percentage by weight of the inorganic base is 4 wt% or greater, preferably 4.5 wt% or greater, and more preferably 5 wt% or greater;
   or, comprising: (a) a N-long-chain acyl amino acid, (e) an inorganic base, and (c) water, wherein the percentage by weight of the N-long-chain acyl amino acid is 30 wt% or greater, preferably 33 wt% or greater, and more preferably 35 wt% or greater, and the molar ratio of the N-long-chain acyl amino acid to the inorganic base is 1:0.98 to 1:0.85, preferably 1:0.96 to 1:0.88, and more preferably 1:0.95 to 1:0.90.
[4] The gel composition according to any one of [1]-[3], wherein the N-long-chain acyl group in the N-long-chain acyl amino acid is derived from a saturated or unsaturated linear or branched fatty acid with 8-22 carbon atoms;
   and/or, the amino acid in the N-long-chain acyl amino acid is derived from one or more of glycine, alanine, glutamic acid, sarcosine, aspartic acid, leucine, isoleucine, valine, threonine, proline, phenylalanine, arginine, lysine, or (methyl) taurine.
[5] The gel composition according to any one of [1]-[4], wherein the N-long-chain acyl group in the N-long-chain acyl amino acid is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl;
   and/or, the amino acid in the N-long-chain acyl amino acid is derived from one or more of alanine, glycine, glutamic acid, sarcosine, arginine, lysine, or (methyl) taurine, preferably alanine, and most preferably L-alanine.
[6] The gel composition according to any one of [1]-[5], wherein the N-long-chain acyl amino acid is selected from one or more of cocoyl alanine, lauroyl alanine, lauroyl sarcosine, cocoyl sarcosine, cocoyl glutamic acid, lauroyl glutamic acid, oleoyl glutamic acid, cocoyl glycine, lauroyl glycine, and stearoyl glutamic acid, preferably lauroyl alanine, and more preferably N-lauroyl-L-alanine.
[7] The gel composition according to any one of [1]-[6], wherein the basic amino acid is selected from one or more of arginine, lysine, citrulline, ornithine, creatine, histidine, diaminobutyric acid, and diaminopropionic acid, preferably arginine and/or lysine, and most preferably arginine;
   and/or, the inorganic base is selected from one or more of sodium hydroxide and potassium hydroxide, preferably, sodium hydroxide.
[8] The gel composition according to any one of [1]-[7], wherein the N-long-chain acyl group in the N-long-chain acyl amino acid dipeptide is derived from a saturated or unsaturated linear or branched fatty acid with 8-22 carbon atoms; preferably, the N-long-chain acyl group in the N-long-chain acyl amino acid dipeptide is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl;
   and/or, the amino acids in the N-long-chain acyl amino acid dipeptide are derived from one or more of alanine, glycine, glutamic acid, sarcosine, arginine, lysine, or (methyl) taurine, preferably L-alanine.
[9] The gel composition according to any one of [1]-[8], wherein the N-long-chain acyl amino acid dipeptide is selected from one or more of long-chain acyl glycyl glycine, long-chain acyl glutamyl glutamic acid, long-chain acyl alanyl alanine, and long-chain acyl sarcosyl sarcosine.
[10] The gel composition according to any one of [2] and [4]-[9], wherein the percentage by weight of the N-long-chain acyl amino acid is 55 wt% or less, preferably 50 wt% or less, and more preferably 45 wt% or less.
[11] The gel composition according to any one of [3]-[9], wherein the percentage by weight of the N-long-chain acyl amino acid is 75 wt% or less, preferably 65 wt% or less, and more preferably 60 wt% or less.
[12] The gel composition according to any one of [1]-[11], wherein the percentage by weight of the N-long-chain acyl amino acid dipeptide is 0.5 wt% or greater, preferably 0.75 wt% or greater, and more preferably 1 wt% or greater;
   and/or, the mass ratio of the N-long-chain acyl amino acid to the N-long-chain acyl amino acid dipeptide is 20:1 to 1.5:1, preferably 15:1 to 3:1, and more preferably 10:1 to 6:1.
[13] The gel composition according to any one of [1]-[12], wherein the basic amino acid or the inorganic base is present in an amount sufficient to partially or completely neutralize the N-long-chain acyl amino acid and N-long-chain acyl amino acid dipeptide, preferably with a degree of neutralization of 85% or greater, more preferably a degree of neutralization of 88% or 90% or greater, and less than 100%; preferably with a degree of neutralization of 85%-98%, 88%-96%, more preferably 90%-95%.
[14] The gel composition according to any one of [1]-[13], wherein the composition is free or substantially free of a thickening agent, preferably completely free of a thickening agent;
   and/or, the composition is free or substantially free of sodium chloride.
[15] The gel composition according to [14], wherein the thickening agent is selected from one or more of cellulose, a mucopolysaccharide, polyvinyl alcohol, a gum, sodium polyacrylate, polyvinyl pyrrolidone, and a vinyl polymer.
[16] The gel composition according to any one of [1]-[15], wherein the gel composition has a pH value of 4.5 or greater, preferably 5 or greater, and more preferably 5.5 or greater, and 12 or less, preferably 11 or less, and more preferably 10 or less, as measured by the dilution method specified in GB/T 13531.1.
[17] The gel composition according to any one of [1]-[16], wherein the composition, after forming a gel, has a hardness of 1.80 or less, preferably 1.50 or less, and more preferably 1.00 or less, as measured by RHEOTECH (Japan) durometer in the conditions of a load of 2 kg, an adapter of 20 mmφ, a penetration speed of 6 cm/min, and a penetration distance of 10 mm.
[18] The gel composition according to any one of [1]-[17], wherein the composition further comprises a water-soluble grease and/or an alkyl polyglycoside.
[19] The gel composition according to claim [18], wherein the water-soluble grease is selected from one or more of a PEG olive oil ester, a PEG isostearate, a PEG cocoyl ester, and a PEG avocado oil ester; preferably one or more of a PEG-10 olive oil glyceride, an olive oil PEG-7 ester, an olive oil PEG-8 ester, a PEG-7 olive oil ester, a PEG-15 glyceryl isostearate, a PEG-9 cocoglyceride, and a PEG-11 avocado glyceride.
[20] A gel, wherein the gel is formed on the basis of the self-thickening of a N-long-chain acyl amino acid and a basic amino acid;
   or, on the basis of the self-thickening of a N-long-chain acyl amino acid, a basic amino acid, and a N-long-chain acyl amino acid dipeptide;
   or, on the basis of the self-thickening of a N-long-chain acyl amino acid and an inorganic base;
   or, on the basis of the self-thickening of a N-long-chain acyl amino acid, an inorganic base, and a N-long-chain acyl amino acid dipeptide.
[21] A gel, wherein the gel is prepared from the gel composition according to any one of [1]-[19].
[22] The gel according to [20] or [21], wherein the gel can be used normally after being kept at 40 ± 1 °C for 24 hours and returned to room temperature;
   and/or, the gel can be used normally after being kept at -8 ± 2 °C for 24 hours and returned to room temperature.
[23] The gel according to any one of claims [20]-[22], wherein the gel is a cleansing gel, a skin care gel, a shampoo gel, a body wash gel, a shaving gel, a makeup remover gel, a massage gel, a make-up gel, a cosmetic gel, or a multifunctional gel.
[24] Use of the gel composition according to any one of [1]-[19] in a cleansing composition, a detergent composition, a cosmetic composition, a skin care composition, or an oral care composition.
[25] A toothpaste, comprising the gel composition according to any one of [1]-[19].
[26] A cosmetic product, comprising the gel composition according to any one of [1]-[19].
[27] A cleansing product, comprising the gel composition according to any one of [1]-[19].
[28] A method for preparing the gel composition according to any one of [1]-[19], comprising:
   mixing (a), (b) or (e), (c), and optionally (d) at 75-90 °C, preferably 80-85 °C,
   wherein preferably, (b) or (e) and (c) are first mixed to give solution I, then (a) and optionally (d) are melted at 75-90 °C, preferably 80-85 °C, to give solution II, and finally the solution I and solution II are mixed at 75-90 °C, preferably 80-85 °C.
[29] A method for imparting gel characteristics to a cleansing composition, wherein the cleansing composition comprises (a) a N-long-chain acyl amino acid and (b) a basic amino acid; the percentage by weight of the N-long-chain acyl amino acid is 30 wt% or greater, preferably 33 wt% or greater, and more preferably 35 wt% or greater, and the percentage by weight of the basic amino acid is 15 wt% or greater, preferably 17 wt% or greater, and more preferably 20 wt% or greater;
   or, the cleansing composition comprises (a) a N-long-chain acyl amino acid, (b) a basic amino acid, and (d) a N-long-chain acyl amino acid dipeptide; the sum of the percentages by weight of the N-long-chain acyl amino acid and N-long-chain acyl amino acid dipeptide is 20 wt% or greater, preferably 23 wt% or greater, and more preferably 25 wt% or greater, and the percentage by weight of the basic amino acid is 10 wt% or greater, preferably 12 wt% or greater, and more preferably 14 wt% or greater;
   or, the cleansing composition comprises (a) a N-long-chain acyl amino acid and (b) a basic amino acid; the percentage by weight of the N-long-chain acyl amino acid is 20 wt% or greater, preferably 23 wt% or greater, and more preferably 25 wt% or greater, and the molar ratio of the N-long-chain acyl amino acid to the basic amino acid is 1:0.98 to 1:0.85, preferably 1:0.96 to 1:0.88, and more preferably 1:0.95 to 1:0.90.
[30] A method for imparting gel characteristics to a cleansing composition, wherein the cleansing composition comprises (a) a N-long-chain acyl amino acid and (e) an inorganic base; the percentage by weight of the N-long-chain acyl amino acid is 40 wt% or greater, preferably 43 wt% or greater, and more preferably 45 wt% or greater, and the percentage by weight of the inorganic base is 5.5 wt% or greater, preferably 6 wt% or greater, and more preferably 6.5 wt% or greater;
   or, the cleansing composition comprises (a) a N-long-chain acyl amino acid, (e) an inorganic base, and (d) a N-long-chain acyl amino acid dipeptide; the sum of the percentages by weight of the N-long-chain acyl amino acid and N-long-chain acyl amino acid dipeptide is 30 wt% or greater, preferably 33 wt% or greater, and more preferably 35 wt% or greater, and the percentage by weight of the inorganic base is 4 wt% or greater, preferably 4.5 wt% or greater, and more preferably 5 wt% or greater;
   or, the cleansing composition comprises (a) a N-long-chain acyl amino acid and (e) an inorganic base; the percentage by weight of the N-long-chain acyl amino acid is 30 wt% or greater, preferably 33 wt% or greater, and more preferably 35 wt% or greater, and the molar ratio of the N-long-chain acyl amino acid to the inorganic base is 1:0.98 to 1:0.85, preferably 1:0.96 to 1:0.88, and more preferably 1:0.95 to 1:0.90.
[31] The method according to [29] or [30], wherein the N-long-chain acyl group in the N-long-chain acyl amino acid is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl;
   and/or, the amino acid in the N-long-chain acyl amino acid is derived from one or more of alanine, glycine, glutamic acid, sarcosine, arginine, lysine, or (methyl) taurine, preferably alanine, and most preferably L-alanine.
[32] The method according to any one of [29]-[31], wherein the basic amino acid is selected from one or more of arginine, lysine, citrulline, ornithine, creatine, histidine, diaminobutyric acid, and diaminopropionic acid, preferably arginine and/or lysine, and most preferably arginine;
   and/or, the inorganic base is selected from one or more of sodium hydroxide and potassium hydroxide, preferably, sodium hydroxide.
[33] The method according to any one of [29]-[32], wherein the N-long-chain acyl group in the N-long-chain acyl amino acid dipeptide is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl;
   and/or, the amino acids in the N-long-chain acyl amino acid dipeptide are derived from one or more of alanine, glycine, glutamic acid, sarcosine, arginine, lysine, or (methyl) taurine, preferably alanine, and most preferably L-alanine;
   preferably, the N-long-chain acyl amino acid dipeptide is selected from one or more of long-chain acyl glycyl glycine, long-chain acyl glutamyl glutamic acid, long-chain acyl alanyl alanine, and long-chain acyl sarcosyl sarcosine.
[34] The method according to [29], wherein the percentage by weight of the N-long-chain acyl amino acid is 55 wt% or less, preferably 50 wt% or less, and more preferably 45 wt% or less.
[35] The method according to [30], wherein the percentage by weight of the N-long-chain acyl amino acid is 75 wt% or less, preferably 65 wt% or less, and more preferably 60 wt% or less.
[36] The method according to any one of [29]-[35], wherein the percentage by weight of the N-long-chain acyl amino acid dipeptide is 0.5 wt% or greater, preferably 0.75 wt% or greater, and more preferably 1 wt% or greater; and/or, the mass ratio of the N-long-chain acyl amino acid to the N-long-chain acyl amino acid dipeptide is 20:1 to 1.5:1, preferably 15:1 to 3:1, and more preferably 10:1 to 6:1.
[37] The method according to any one of [29]-[36], wherein the composition is free or substantially free of a thickening agent; and/or, the composition is free or substantially free of sodium chloride.
[38] The method according to any one of [29]-[37], wherein the basic amino acid or the inorganic base is present in an amount sufficient to partially or completely neutralize the N-long-chain acyl amino acid and N-long-chain acyl amino acid dipeptide, preferably with a degree of neutralization of 85% or greater, more preferably a degree of neutralization of 88% or greater, and less than 100%.
[39] According to any one of the preceding items, wherein the composition consists of (a) a N-long-chain acyl amino acid, (b) a basic amino acid, (c) water, and optionally, (d) a N-long-chain acyl amino acid dipeptide; or, the composition consists of (a) a N-long-chain acyl amino acid, (e) an inorganic base, (c) water, and optionally, (d) a N-long-chain acyl amino acid dipeptide; or, the composition consists of (a) a N-long-chain acyl amino acid, (b) a basic amino acid/(e) an inorganic base, (c) water, and optionally, (d) a N-long-chain acyl amino acid dipeptide, and a preservative and/or a sweetener.

Compared with the prior art, the present disclosure has the following beneficial technical effects:
1. The gel composition/gel product of the present disclosure takes an amino acid surfactant (component (a)) as the primary surfactant/sole surfactant; particularly when compounded with the basic amino acid (component (b)), the gel composition/gel product is environment-friendly, nontoxic and harmless, and mild to human skin in use and non-irritant, and can reduce the dependence on petroleum-based products.
2. The present disclosure prepares gel products without using conventional thickening agents/gelling agents, and can avoid the influence of the thickening agent/gelling agent on the product performance (color, foam, efficacy, user experience, and the like).
3. The inventor surprisingly found that, the addition of N-long-chain acyl amino acid dipeptide can greatly reduce the amount of raw materials (that is, the gel can be formed with a smaller amount of N-long-chain acyl amino acid), and can improve the thermo- and cryo-resistance of the gel to a certain extent and improve the extrusion and truncation performance of the gel.
4. The inventor also surprisingly found that, when the degree of neutralization is 85% or greater and less than 100% (i.e., the N-long-chain acyl amino acid/dipeptide is not completely neutralized, and the system contains a small amount of N-long-chain acyl amino acid/dipeptide), the consumption of raw materials can be greatly reduced, and a softer gel with improved extrusion and truncation performance may be obtained.
5. The gel cleansing product features good portability and is suitable for various non-domestic circumstances such as outdoors, in supermarkets, and the like.
6. The gel formed by compounding (a) a N-long-chain acyl amino acid, (b) a basic amino acid, and optional (d) a N-long-chain acyl amino acid dipeptide features multifunctionality and good cleansing capability, and can be used as a hand wash product, a body wash product, a shampoo product, a makeup remover product, or a toothpaste, as well as a detergent for tableware and the like outdoors.

### BRIEF DESCRIPTION OF THE DRAWINGS

FIG. 1 illustrates the appearance of the gels of Examples 1-7, wherein panels a-g show the appearance of the gels of Examples 1-7, respectively;
FIG. 2 illustrates the appearance of the gels of Examples 8-10, wherein panels a-g show the appearance of the gels of Examples 8-10, respectively (panel a shows the appearance of the gel of Example 8, panel b shows the appearance of the gel of Example 9, and panel c shows the appearance of the gel of Example 10);
FIG. 3 illustrates the appearance of the gels of Examples 11 and 12, wherein panel a shows the appearance of the gel of Example 11, and panel b shows the appearance of the gel of Example 12;
FIG. 4 illustrates the appearance of the gels of Examples 13-15, wherein panels a-c show the appearance of the gels of Examples 13-15, respectively; and
FIG. 5 illustrates the appearance of the gel of Example 16.

### DETAILED DESCRIPTION

In one embodiment, provided is a gel composition. The composition comprises (a) a N-long-chain acyl amino acid, (b) a basic amino acid, and (c) water.

In component (a), the N-long-chain acyl group in the N-long-chain acyl amino acid is derived from a saturated or unsaturated linear or branched fatty acid with 8-22 carbon atoms. Furthermore, the N-long-chain acyl group in the N-long-chain acyl amino acid is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl.

Furthermore, the amino acid in the N-long-chain acyl amino acid is derived from one or more of glycine, alanine, glutamic acid, sarcosine, aspartic acid, leucine, isoleucine, valine, threonine, proline, phenylalanine, arginine, or lysine. Furthermore, the amino acid in the N-long-chain acyl amino acid is derived from one or more of alanine, glycine, glutamic acid, sarcosine, arginine, lysine, or (methyl) taurine, preferably alanine, and most preferably L-alanine.

The N-long-chain acyl (methyl) taurine described herein refers to a N-long-chain acyl methyl taurine or a N-long-chain acyl taurine.

As an example, the N-long-chain acyl amino acid may be selected from cocoyl alanine, lauroyl alanine, cocoyl sarcosine, lauroyl sarcosine, lauroyl glutamic acid, cocoyl glutamic acid, oleoyl glutamic acid, cocoyl glycine, lauroyl glycine, stearoyl glutamic acid, and the like, preferably lauroyl alanine and lauroyl sarcosine, and more preferably N-lauroyl-L-alanine. A salt of the relevant N-long-chain acyl amino acid may also be selected as the starting material. Those skilled in the art will appreciate that, there's no essential difference between the use of the salt or the use of the corresponding acid as the starting material, and the two are equivalent technical features.

For the synthesis process of N-long-chain acyl amino acids, one may refer to the general synthesis methods for N-acyl amino acid surfactant, including direct synthesis and indirect synthesis. The direct synthesis methods using fatty acid raw materials include enzymatic synthesis and dehydration synthesis. The indirect synthesis methods include fatty acid chloride acylation, aliphatic nitrile acylation hydrolysis, fatty acid anhydride acylation, amide carbonylation, and the like. One preferred method is the reaction between a fatty acid chloride and the amino group of an amino acid (Shotten-Baumann condensation or Shotten-Baumann reaction).

A typical preparation process suitable for the present disclosure comprises, for example: dissolving an amino acid and sodium hydroxide in water or a mixed solution of water and acetone to give an amino acid salt solution; slowly and dropwise adding lauroyl chloride and a sodium hydroxide solution into the amino acid salt solution, controlling the pH value of the reaction system, and performing post-treatment on the product after the dropwise addition. Representative methods are disclosed in CN1798821A, US6703517B2, CN102875409B, JPH0570418A, etc.

For the post-treatment of the N-long-chain acyl amino acid product, conventional methods such as recrystallization, water rinsing, drying, etc., may be used. One preferred post-treatment comprises: mixing the crude N-long-chain acyl amino acid product with a solvent, optionally stirring, and controlling the temperature T of the mixture system in a range of the melting point of the long-chain fatty acid to the melting point of the N-long-chain acyl amino acid, wherein the solvent is water, an organic solvent, or a mixed solution of water and an organic solvent; and after the temperature control in the system, separating the solid and liquid phases. Related methods are disclosed by the inventor in CN202210867760.7 or PCT/CN2022/107270, the relevant content of which is incorporated herein.

For component (b), the basic amino acid is selected from one or more of arginine, lysine, citrulline, ornithine, creatine, histidine, diaminobutyric acid, and diaminopropionic acid, preferably arginine and/or lysine, and most preferably arginine. On one hand, arginine is beneficial to the thermo- and cryo-resistance and extrusion performance of the gel product; on the other hand, for oral gels, arginine has the capability to promote the deposition of calcium and phosphate ions on the surface of dentin and dentin tubules and the functionality of isolating external stimulation and delaying dentin sensitivity.

For components (a) and (b) above, the basic amino acid salt of the N-long-chain acyl amino acid may also be directly added, and it has no essential difference from the separate addition of components (a) and (b) and shall also fall within the scope of the present disclosure.

Component (c) is not specified. Specifically, ion-exchanged water, distilled water, purified water, river water, well water, natural water, underground water, city water, hard water, soft water, or the like may be used. One of these may be used, or two or more selected from the above group may be used in combination. From the viewpoint of storage stability and hygiene of the product of the present disclosure, ion-exchanged water, distilled water, and purified water are preferred.

The percentage by weight of component (a), the N-long-chain acyl amino acid, used in this embodiment is 30 wt% or greater, preferably 31 wt%, 32 wt%, 33 wt%, 34 wt%, or 35 wt% or greater. An insufficient amount may be difficult to give a gel. Unless otherwise specified, all percentages by weight in the present disclosure are relative to the composition.

The percentage by weight of component (a), the N-long-chain acyl amino acid, is 55 wt% or less, preferably 54 wt%, 53 wt%, 52 wt%, 51 wt%, 50 wt%, 49 wt%, 48 wt%, 47 wt%, 46 wt%, 45 wt%, 44 wt%, 43 wt%, 42 wt%, 41 wt%, or 40 wt% or less. An insufficient amount may lead to a hard gel, which is undesirable for extrusion and truncation.

The percentage by weight of component (b), the basic amino acid, is 15 wt% or greater, preferably 16 wt%, 17 wt%, 18 wt%, 19 wt%, 20 wt%, 21 wt%, or 22 wt% or greater.

In one embodiment, provided is a gel composition. The composition comprises: (a) a N-long-chain acyl amino acid, (b) a basic amino acid, and (c) water, wherein the percentage by weight of the N-long-chain acyl amino acid is 20 wt% or greater, preferably 23 wt% or greater, and more preferably 25 wt% or greater. Please refer to other embodiments for the description of components (a), (b), and (c).

Preferably, the basic amino acid is present in an amount sufficient to partially or completely neutralize the N-long-chain acyl amino acid. In one preferred embodiment, the degree of neutralization is 85% or greater, more preferably above 88% or greater. For example, the degree of neutralization is 86%, 87%, 88%, 89%, 90%, 91%, 92%, 93%, 94%, 95%, 96%, 97%, or 98%.

Furthermore, the molar ratio of the N-long-chain acyl amino acid to the basic amino acid is 1:0.98 to 1:0.85, preferably 1:0.96 to 1:0.88, and more preferably 1:0.95 to 1:0.90. Illustratively, when the N-long-chain acyl amino acid is present at 1 mol, the basic amino acid is present at 0.98, 0.97, 0.96, 0.95, 0.94, 0.93, 0.92, 0.91, 0.90, 0.89, 0.88, 0.87, 0.86, or 0.85 mol.

The inventor surprisingly found that, when the degree of neutralization is 85% or greater and less than 100%, a small amount of the N-long-chain acyl amino acid and/or N-long-chain acyl amino acid dipeptide that is not completely neutralized may be present in the composition system, which can greatly reduce the consumption of raw materials and give a softer gel with improved extrusion and truncation performance may be obtained. However, excessive N-long-chain acyl amino acid and/or N-long-chain acyl amino acid dipeptide residues may adversely affect the stability of the system, and the excessive N-long-chain acyl amino acid may be gradually precipitated or lead to whitening in the use of the product.

In one embodiment, provided is a gel composition. The composition comprises: (a) a N-long-chain acyl amino acid, (b) a basic amino acid, (c) water, and (d) a N-long-chain acyl amino acid dipeptide.

Please refer to other embodiments for the description of components (a), (b), and (c), which is not recited herein. Component (d), the N-long-chain acyl amino acid dipeptide, is a known concept, and refers to a long-chain acyl aminoacyl amino acid, typically, for example, long-chain acyl glycyl glycine, long-chain acyl glutamyl glutamic acid, long-chain acyl alanyl alanine, long-chain acyl sarcosyl sarcosine, and the like, as described in, e.g., CN100448968C, PCT/CN2022/107270, etc.

The N-long-chain acyl amino acid dipeptide may be prepared from an acid chloride derived from an N-long-chain acyl amino acid and an amino acid by the Schotten-Baumann reaction or the like, or by the reaction of an amino acid dipeptide (such as glutamyl glutamic acid) and an acid chloride. One preferred relevant preparation method is disclosed in PCT/CN2022/107270, the relevant content of which is incorporated herein.

The N-long-chain acyl group in the N-long-chain acyl amino acid dipeptide is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl.

The amino acids in the N-long-chain acyl amino acid dipeptide are derived from one or more of alanine, glycine, glutamic acid, sarcosine, arginine, lysine, or (methyl) taurine, preferably L-alanine. A N-long-chain acyl-L-alanine dipeptide, particularly, lauroyl alanyl alanine, is preferred in the present disclosure.

The addition of N-long-chain acyl amino acid dipeptide can greatly reduce the amount of raw materials (that is, the gel can be formed with a smaller amount of N-long-chain acyl amino acid), and can improve the thermo- and cryo-resistance of the gel to a certain extent and improve the extrusion and truncation performance of the gel.

Preferably, the sum of the percentages by weight of the N-long-chain acyl amino acid and the N-long-chain acyl amino acid dipeptide is 20 wt% or greater, preferably 21 wt%, 22 wt%, 23 wt%, 24 wt%, 25 wt%, 26 wt%, 27 wt%, or 28 wt% or greater. An insufficient amount may be difficult to give a gel. The specific addition amount may be adjusted mainly depending on the content of the N-long-chain acyl amino acid dipeptide, and a higher content of the N-long-chain acyl amino acid dipeptide may lead to a lower sum of the percentages by weight of the corresponding N-long-chain acyl amino acid and the N-long-chain acyl amino acid dipeptide.

The percentage by weight of the N-long-chain acyl amino acid dipeptide is 0.5 wt% or greater, preferably 0.75 wt%, 1 wt%, 1.5 wt%, 2 wt% or greater.

The mass ratio of the N-long-chain acyl amino acid to the N-long-chain acyl amino acid dipeptide is 20:1 to 1.5:1, preferably 15:1 to 3:1, and more preferably 10:1 to 6:1. The mass ratio of the N-long-chain acyl amino acid to the N-long-chain acyl amino acid dipeptide is, for example, 20, 19, 18, 17, 16, 15, 14, 13, 12, 11, 10, 9, 8, 7, 6, 5, 4, or 3.

The percentage by weight of component (a), the N-long-chain acyl amino acid, is 55 wt% or less, preferably 54 wt%, 53 wt%, 52 wt%, 51 wt%, 50 wt%, 49 wt%, 48 wt%, 47 wt%, 46 wt%, 45 wt%, 44 wt%, 43 wt%, 42 wt%, 41 wt%, or 40 wt% or less. An insufficient amount may lead to a hard gel, which is undesirable for extrusion and truncation.

The percentage by weight of component (b), the basic amino acid, is 10 wt% or greater, preferably 11 wt%, 12 wt%, 13 wt%, 14 wt%, 15 wt%, 16 wt%, or 17 wt% or greater. Preferably, the basic amino acid is present in an amount sufficient to partially or completely neutralize the N-long-chain acyl amino acid and the N-long-chain acyl amino acid dipeptide, for example, as can be seen in the addition amounts in the preceding embodiments. When the neutralization degree is less than 100% and the composition contains the N-long-chain acyl amino acid dipeptide, the amount of raw material (specifically, the N-long-chain acyl amino acid and the dipeptide) can be minimized, and the hardness of the product is relatively minimized, thus facilitating the packaging of the gel.

In one embodiment, provided is a gel composition. The composition comprises: (a) a N-long-chain acyl amino acid, (e) an inorganic base, and (c) water.

Please refer to other embodiments for the description of components (a) and (c). Component (e), the inorganic base, is selected from one or more of sodium hydroxide and potassium hydroxide, preferably, sodium hydroxide. In general, conventional sodium hydroxide can be used without specific limitation. If a sodium hydroxide solution is used, the amount refers to the amount of sodium hydroxide, rather than the amount of the sodium hydroxide solution.

For components (a) and (e) above, the corresponding salt of the N-long-chain acyl amino acid may also be directly added, and it has no essential difference from the separate addition of components (a) and (e) and shall also fall within the scope of the present disclosure.

The percentage by weight of component (a), the N-long-chain acyl amino acid, is 40 wt% or greater, preferably 43 wt% or greater, and more preferably 45 wt% or greater. An insufficient amount may be difficult to give a gel.

The percentage by weight of component (a), the N-long-chain acyl amino acid, is 75 wt% or less, preferably 70 wt%, 68 wt%, 65 wt%, 64 wt%, 63 wt%, 62 wt%, 61 wt%, 60 wt%, 59 wt%, 58 wt%, 57 wt%, 56 wt%, or 55 wt% or less. An insufficient amount may lead to a hard gel, which is undesirable for extrusion and truncation.

The percentage by weight of component (e), the inorganic base, is 5.5 wt% or greater, preferably 6 wt% or greater, and more preferably 6.5 wt% or greater. Preferably, the inorganic base is present in an amount sufficient to partially or completely neutralize the N-long-chain acyl amino acid.

In one embodiment, provided is a gel composition. The composition comprises: (a) a N-long-chain acyl amino acid, (e) an inorganic base, and (c) water, wherein the percentage by weight of the N-long-chain acyl amino acid is 30 wt% or greater, preferably 33 wt% or greater, and more preferably 35 wt% or greater.

Preferably, the inorganic base is present in an amount sufficient to partially or completely neutralize the N-long-chain acyl amino acid. In one preferred embodiment, the degree of neutralization is 85% or greater, more preferably above 88% or greater. For example, the degree of neutralization is 86%, 87%, 88%, 89%, 90%, 91%, 92%, 93%, 94%, 95%, 96%, 97%, or 98%.

Furthermore, the molar ratio of the N-long-chain acyl amino acid to the inorganic base is 1:0.98 to 1:0.85, preferably 1:0.96 to 1:0.88, and more preferably 1:0.95 to 1:0.90. Illustratively, when the N-long-chain acyl amino acid is present at 1 mol, the inorganic base is present at 0.98, 0.97, 0.96, 0.95, 0.94, 0.93, 0.92, 0.91, 0.90, 0.89, 0.88, 0.87, 0.86, or 0.85 mol.

The inventor surprisingly found that, when the degree of neutralization is 85% or greater and less than 100%, a small amount of the N-long-chain acyl amino acid and/or N-long-chain acyl amino acid dipeptide may be present in the composition system, which can greatly reduce the consumption of raw materials and give a softer gel with improved extrusion and truncation performance may be obtained.

In one embodiment, provided is a gel composition. The composition comprises: (a) a N-long-chain acyl amino acid, (e) an inorganic base, (c) water, and (d) a N-long-chain acyl amino acid dipeptide. Please refer to other embodiments for the description of components (a), (e), (c), and (d).

The addition of N-long-chain acyl amino acid dipeptide can greatly reduce the amount of raw materials (that is, the gel can be formed with a smaller amount of N-long-chain acyl amino acid), and can improve the thermo- and cryo-resistance of the gel to a certain extent and improve the extrusion and truncation performance of the gel.

Preferably, the sum of the percentages by weight of the N-long-chain acyl amino acid and the N-long-chain acyl amino acid dipeptide is 30 wt% or greater, preferably 31 wt%, 32 wt%, 33 wt%, 34 wt%, 35 wt%, 36 wt%, 37 wt%, 38 wt%, or 39 wt% or greater. An insufficient amount may be difficult to give a gel. The specific addition amount may be adjusted mainly depending on the content of the N-long-chain acyl amino acid dipeptide, and a higher content of the N-long-chain acyl amino acid dipeptide may lead to a lower sum of the percentages by weight of the corresponding N-long-chain acyl amino acid and the N-long-chain acyl amino acid dipeptide.

The percentage by weight of the N-long-chain acyl amino acid dipeptide is 0.5 wt% or greater, preferably 0.75 wt%, 1 wt%, 1.5 wt%, 2 wt% or greater.

The mass ratio of the N-long-chain acyl amino acid to the N-long-chain acyl amino acid dipeptide is 20:1 to 1.5:1, preferably 15:1 to 3:1, and more preferably 10:1 to 6:1. The mass ratio of the N-long-chain acyl amino acid to the N-long-chain acyl amino acid dipeptide is, for example, 20, 19, 18, 17, 16, 15, 14, 13, 12, 11, 10, 9, 8, 7, 6, 5, 4, or 3.

The percentage by weight of component (a), the N-long-chain acyl amino acid, is 75 wt% or less, preferably 70 wt%, 68 wt%, 65 wt%, 64 wt%, 63 wt%, 62 wt%, 61 wt%, 60 wt%, 59 wt%, 58 wt%, 57 wt%, 56 wt%, or 55 wt% or less. An insufficient amount may lead to a hard gel, which is undesirable for extrusion and truncation.

The percentage by weight of component (e), the inorganic base, is 4 wt% or greater, preferably 4.5 wt% or greater, and more preferably 5 wt% or greater. Preferably, the inorganic base is present in an amount sufficient to partially or completely neutralize the N-long-chain acyl amino acid and the N-long-chain acyl amino acid dipeptide.

In various embodiments, relevant teachings of other embodiments may also be referred to for the details that are not discussed, provided that the contents do not conflict.

In various embodiments, the compositions of the present disclosure are free or substantially free of a thickening agent. The "substantially free of" refers to that the amount of thickening agent in the composition does not significantly affect the performance of the product and the amount of thickening agent is insufficient to form a gel, i.e., the gel formation in the present disclosure does not result from the action of the thickening agent. Preferably, the composition of the present disclosure is completely free of conventional thickening agents. As described in the Background, conventional thickening agents/gelling agents adversely affect product performance (color, foam, efficacy, user experience, and the like).

Furthermore, the composition of the present disclosure does not contain a thickening agent such as cellulose, mucopolysaccharides, polyvinyl alcohol, gums, sodium polyacrylate, polyvinyl pyrrolidone, vinyl polymers, or the like. For example, the composition of the present disclosure does not contain starch, gelatin, sodium alginate, guar gum, chitin gum, gum arabic, xanthan gum, soy protein gum, polyacrylamide, polyvinyl alcohol, polyvinylpyrrolidone, modified paraffin resin, carbomer resin, styrene butadiene rubber, or the like.

Furthermore, the composition of the present disclosure does not involve an AES system or require sodium chloride for thickening, such that the composition is free or substantially free of sodium chloride. The "substantially free of" refers to that the amount of sodium chloride in the composition does not significantly affect the performance of the product and the amount of sodium chloride is insufficient to form a gel.

In various embodiments, a pH of the gel composition of the present disclosure that falls from weakly acidic to weakly basic may facilitate the applications. Specifically, the gel composition has a pH value of 4.5 or greater, preferably 5 or greater, and more preferably 5.5 or greater, and 12 or less, preferably 11 or less, and more preferably 10 or less, as measured by the dilution method specified in GB/T 13531.1.

In various embodiments, the gel composition of the present disclosure, after forming a gel, has a hardness of 1.80 or less, preferably 1.70, 1.60, 1.50, 1.40, 1.30, 1.20, 1.10, or 1.00 or less, and more preferably 0.90, 0.80, 0.70, 0.50, 0.40, 0.30, or 0.20 or less, as measured by RHEOTECH (Japan) durometer in the conditions of a load of 2 kg, an adapter of 20 mmφ, a penetration speed of 6 cm/min, and a penetration distance of 10 mm. A lower gel hardness may better facilitate the filling of a tubular product.

The present disclosure further provides a gel prepared from the gel composition described above. Preferably, the gel of the present disclosure has improved thermo- and cryo-resistance. Specifically, the gel can be used normally after being kept at 40 ± 1 °C for 24 hours and returned to room temperature; the gel can be used normally after being kept at -8 ± 2 °C for 24 hours and returned to room temperature. The term "used normally" refers to no significant changes in appearance and performance of the gel and no influence on use.

The gel of the present disclosure may be used as a cleansing gel, a skin care gel, a shampoo gel, a shaving gel, a makeup remover gel, a massage gel, a make-up gel, a cosmetic gel, or a multifunctional gel. The multifunctionality refers to the versatility of use in a variety of scenes and with a variety of uses. For example, the gel can be used as a hand wash product, a body wash product, a shampoo product, a makeup remover product, or a toothpaste, as well as a detergent for a variety of ware such as tableware and the like outdoors.

The present disclosure further provides a toothpaste, comprising the gel composition described above. As a toothpaste product, the present disclosure may be supplemented with a friction agent, a humectant, a sweetener, an essence, a preservative, a colorant, and the like according to the requirement of product performance.

The present disclosure further provides a cosmetic product, comprising the gel composition described above. As a cosmetic product, the present disclosure may be supplemented with a humectant, an essence, a preservative, a colorant, an oil, a suspended particle, and the like according to the requirement of product performance. The suspended particle is selected from an oil-insoluble solid particle or an oil-immiscible liquid. The oil-insoluble solid particle includes mica, starch, zinc oxide, titanium dioxide, talc powder, and a silicone elastomer. The oil-immiscible liquid includes glycerol, water, and a polyol.

The present disclosure further provides a cleansing product, comprising the gel composition described above. As a cleansing product, the present disclosure may be supplemented with a softening agent, a chelating agent, a preservative, glycerin, an essence, and the like according to the requirement of product performance.

The present disclosure further provides a cleansing product of an extremely simplified formulation, comprising only the (a) N-long-chain acyl amino acid, (b) basic amino acid/(e) inorganic base, and (c) water described above; or, comprising only the (a) N-long-chain acyl amino acid, (b) basic amino acid/(e) inorganic base, (c) water described above, and a necessary preservative and/or sweetener.

The present disclosure provides a method for preparing a gel composition, comprising: mixing (a), (b) or (e), (c), and optionally (d) at 75-90 °C, preferably 80-85 °C.

Preferably, (b) or (e) and (c) are first mixed to give solution I, then (a) and optionally (d) are melted at 75-90 °C, preferably 80-85 °C, to give solution II, and finally the solution I and solution II are mixed at 75-90 °C, preferably 80-85 °C.

The present disclosure further provides a method for imparting gel characteristics to a cleansing composition, wherein the cleansing composition comprises (a) a N-long-chain acyl amino acid and (b) a basic amino acid; the percentage by weight of the N-long-chain acyl amino acid is 30 wt% or greater, preferably 33 wt% or greater, and more preferably 35 wt% or greater, and the percentage by weight of the basic amino acid is 15 wt% or greater, preferably 17 wt% or greater, and more preferably 20 wt% or greater;
or, the cleansing composition comprises (a) a N-long-chain acyl amino acid, (b) a basic amino acid, and (d) a N-long-chain acyl amino acid dipeptide; the sum of the percentages by weight of the N-long-chain acyl amino acid and N-long-chain acyl amino acid dipeptide is 20 wt% or greater, preferably 23 wt% or greater, and more preferably 25 wt% or greater, and the percentage by weight of the basic amino acid is 10 wt% or greater, preferably 12 wt% or greater, and more preferably 14 wt% or greater;
or, the cleansing composition comprises (a) a N-long-chain acyl amino acid and (b) a basic amino acid; the percentage by weight of the N-long-chain acyl amino acid is 20 wt% or greater, preferably 23 wt% or greater, and more preferably 25 wt% or greater, and the molar ratio of the N-long-chain acyl amino acid to the basic amino acid is 1:0.98 to 1:0.85, preferably 1:0.96 to 1:0.88, and more preferably 1:0.95 to 1:0.90.

Please refer to other embodiments for the description of components (a), (b), and (d). When the above components are employed, gel characteristics can be imparted to the cleansing composition.

The present disclosure further provides another method for imparting gel characteristics to a cleansing composition, wherein the cleansing composition comprises (a) a N-long-chain acyl amino acid and (e) an inorganic base; the percentage by weight of the N-long-chain acyl amino acid is 40 wt% or greater, preferably 43 wt% or greater, and more preferably 45 wt% or greater, and the percentage by weight of the inorganic base is 5.5 wt% or greater, preferably 6 wt% or greater, and more preferably 6.5 wt% or greater;
or, the cleansing composition comprises (a) a N-long-chain acyl amino acid, (e) an inorganic base, and (d) a N-long-chain acyl amino acid dipeptide; the sum of the percentages by weight of the N-long-chain acyl amino acid and N-long-chain acyl amino acid dipeptide is 30 wt% or greater, preferably 33 wt% or greater, and more preferably 35 wt% or greater, and the percentage by weight of the inorganic base is 4 wt% or greater, preferably 4.5 wt% or greater, and more preferably 5 wt% or greater;
or, the cleansing composition comprises (a) a N-long-chain acyl amino acid and (e) an inorganic base; the percentage by weight of the N-long-chain acyl amino acid is 30 wt% or greater, preferably 33 wt% or greater, and more preferably 35 wt% or greater, and the molar ratio of the N-long-chain acyl amino acid to the inorganic base is 1:0.98 to 1:0.85, preferably 1:0.96 to 1:0.88, and more preferably 1:0.95 to 1:0.90.

Please refer to other embodiments for the description of components (a), (e), and (d). When the above components are employed, gel characteristics can be imparted to the cleansing composition.

The present disclosure will be further illustrated by the following examples in conjunction with the accompanying drawings. It will be appreciated that these examples are merely intended to illustrate the present disclosure rather than limit the scope of the present disclosure. Furthermore, it will be appreciated that various changes or modifications of the present disclosure can be made by those skilled in the art after reading the teachings of the present disclosure, and such equivalents also fall within the scope of the appended claims of the present application.

The present disclosure will be further illustrated with reference to the following specific examples.

### Examples

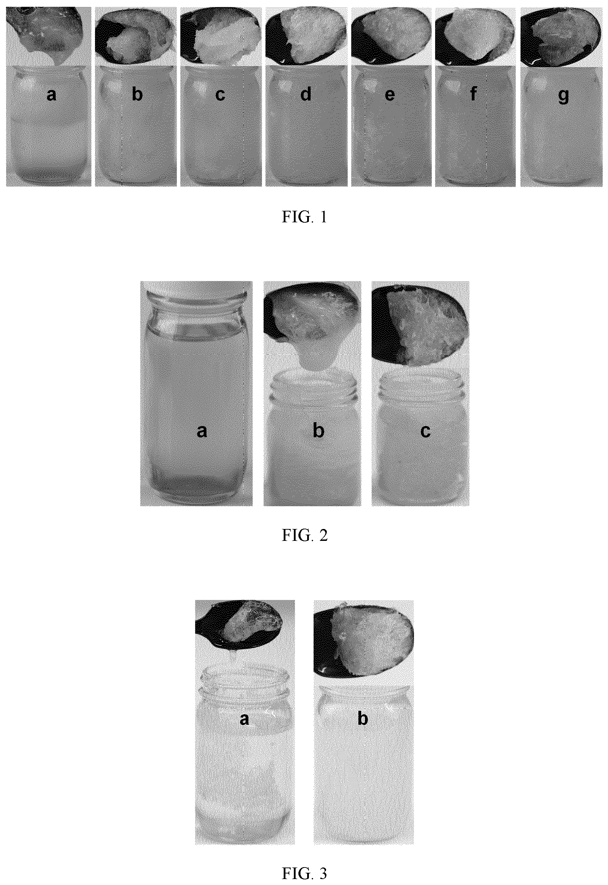

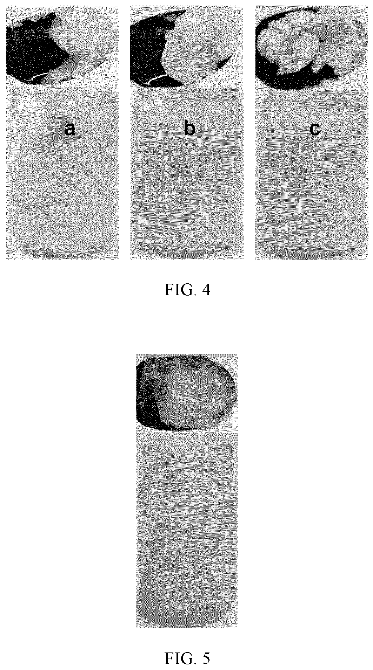

A basic amino acid or sodium hydroxide was mixed with water to give solution I; a N-long-chain acyl amino acid (N-long-chain acyl amino acid dipeptide) was melted at 85 °C to give solution II; finally, solution I was gradually added to solution II at 85 °C with stirring to finally form a gel (or a solution in some examples). The results are detailed in Table 1. The appearance of the gels of Examples 1-7 is shown in FIG. 1; the appearance of the gels of Examples 8-10 is shown in FIG. 2; the appearance of the gels of Examples 11 and 12 is shown in FIG. 3; the appearance of the gels of Examples 13-15 is shown in FIG. 4; the appearance of the gel of Example 16 is shown in FIG. 5.

As shown in FIG. 1, when the percentage by weight of N-lauroyl-L-alanine reached 30 wt%, the gelling started; as the percentage by weight of N-lauroyl-L-alanine increased, the amount of gel gradually increased; when the percentage reached 33 wt%, the solution was completely converted into a gel.

As shown in FIG. 2 where arginine was replaced with sodium hydroxide, when the percentage by weight of N-lauroyl-L-alanine was 35 wt%, the product remained a solution; when the percentage by weight of N-lauroyl-L-alanine was 40 wt%, the majority of the solution was converted into a gel with a small amount of remaining solution; when the content of N-lauroyl-L-alanine was further increased, the solution was completely converted into a gel.

As shown in FIG. 3 where N-lauroyl-L-alanine was replaced with lauroyl sarcosine, when the percentage by weight of lauroyl sarcosine was 33 wt%, the gelling started; when the content was increased to 35%, the solution was completely converted into a gel.

As shown in FIG. 4 where arginine was replaced with lysine, a gel was also formed.

As shown in FIG. 5 where lauroyl alanyl alanine (lauroyl alanine dipeptide) was further introduced, when the total content of N-lauroyl-L-alanine and the dipeptide was 25 wt%, a gel was also formed.

Further studies have found that when the degree of neutralization is 85% or greater and less than 100%, the amount of long-chain acyl amino acid can also be greatly reduced and a softer gel may be obtained (see Examples 17-23). When a small amount of dipeptide is added and no neutralizing agent is added (Example 20), the amount of long-chain acyl amino acids can be further reduced.

**Table 1.**

| Example (I) | N-Lauroyl-L-alanine | Arginine | Water | Appearance of gel |
|---|---|---|---|---|
| 1 | 30 wt% | 19.2 wt% | 50.8 wt% | Small amount of pale yellow gel |
| 2 | 31 wt% | 19.8 wt% | 49.2 wt% | Part of pale yellow gel + part of solution |
| 3 | 32 wt% | 20.5 wt% | 47.5 wt% | Pale yellow gel + small amount of solution |
| 4 | 33 wt% | 21.1 wt% | 45.9 wt% | Pale yellow gel |
| 5 | 35 wt% | 22.4 wt% | 42.6 wt% | Pale yellow gel |
| 6 | 38 wt% | 24.3 wt% | 37.7 wt% | Pale yellow gel |
| 7 | 40 wt% | 25.6 wt% | 34.4 wt% | Pale yellow gel |

| Example (II) | N-Lauroyl-L-alanine | Sodium hydroxide | Water | Appearance of gel |
|---|---|---|---|---|
| 8 | 35 wt% | 5.1 wt% | 59.9 wt% | Pale yellow solution |
| 9 | 40 wt% | 5.8 wt% | 54.2 wt% | Pale yellow gel + small amount of solution |
| 10 | 45 wt% | 6.6 wt% | 48.4 wt% | Pale yellow gel |

| Example (III) | Lauroyl sarcosine*¹ | Arginine | Water | Appearance of gel |
|---|---|---|---|---|
| 11 | 33 wt% | 21.1 wt% | 45.9 wt% | Small amount of white gel |
| 12 | 35 wt% | 22.4 wt% | 42.6 wt% | White gel |

| Example (IV) | N-Lauroyl-L-alanine | Lysine*² | Water | Appearance of gel |
|---|---|---|---|---|
| 13 | 35 wt% | 18.6 wt% | 46.4 wt% | Pale yellow gel |
| 14 | 45 wt% | 24.0 wt% | 31.0 wt% | Pale yellow gel |
| 15 | 50 wt% | 26.6 wt% | 23.4 wt% | Pale yellow gel |

| Example (V) | N-Lauroyl-L-alanine + dipeptide*³ | Arginine | Water | Appearance of gel |
|---|---|---|---|---|
| 16 | 25 wt% | 15.4 wt% | 59.6 wt% | Pale yellow gel |

| Example (VI) | N-Lauroyl-L-alanine | Arginine | Molar ratio*⁴ | Appearance of gel |
|---|---|---|---|---|
| 17 | 26 wt% | 14.1 wt% | 1:0.85 | Pale yellow gel + small amount of solution |
| 18 | 26 wt% | 14.6 wt% | 1:0.88 | Pale yellow gel + small amount of solution |
| 19 | 26 wt% | 15.0 wt% | 1:0.90 | Pale yellow gel + small amount of solution |
| 20 | 25 wt%*⁵ | 14.5 wt% | 1:0.90 | Pale yellow gel |
| 21 | 27 wt% | 15.6 wt% | 1:0.90 | Pale yellow gel |
| 22 | 28 wt% | 16.2 wt% | 1:0.90 | Pale yellow gel |
| 23 | 30 wt% | 17.4 wt% | 1:0.90 | Pale yellow gel |

| | | | | |
|---|---|---|---|---|
| *1. Prepared by reacting aqueous lauroyl sarcosine with hydrochloric acid. *2. Prepared by reacting aqueous lysine hydrochloride with sodium hydroxide. *3. The dipeptide refers to lauroyl alanyl alanine, and "N-lauroyl-L-alanine + dipeptide" was prepared according to the method in Example 503# and Example 6 in PCT/CN2022/107270. *4. In Example (VI), the other part than N-lauroyl-L-alanine and arginine was water, and the molar ratio refers to N-lauroyl-L-alanine:arginine. *5. In Example 20, the composition of the long-chain acyl amino acid was: 95% of N-lauroyl-L-alanine + 5% of lauroyl alanine dipeptide. | | | | |

### Performance Evaluation

Sense of touch: determined by 3 independent professional testers.

Hardness: instrument: RHEOTECH (Japan) durometer/rheometer; conditions: load: 2 kg (2K/20N), adapter: 20 mmφ, penetration speed: 6 cm/min, penetration distance: 10 mm.

pH: the pH of the solution was measured by the dilution method specified in GB/T 13531.1.

Thermo-resistance test: the gel was kept for 24 h at 40 °C or for 1 h at 60 °C, 70 °C or 80 °C and returned to room temperature to explore the stability of the gel in high-temperature conditions and whether the gel could be normally used after being returned to the room temperature.

Cryo-resistance test: the gel was kept for 24 h at -6 °C or for 12 h at -18 °C and returned to room temperature to explore the stability of the gel in low-temperature conditions and whether the gel could be normally used after being returned to the room temperature.

Stability test: the gel was observed for one month at -6 °C and 50 °C, in the Cycle test, and in the light resistance test. Cycle test: cycles of 0 °C × 2 days → 25 °C × 2 days → 40 °C × 2 days; light resistance test: fluorescent lamp irradiation conditions: about 23000 Lux/day, 25 °C.

The gel was observed at 40 °C for 3 months, at 5 °C for 3 months, and at room temperature for 3 months.

The results are shown in Table 2.

**Table 2.**

| Example (I) | Sense of touch | Hardness 2K/20N | pH value | Thermo-resistance | Cryo-resistance | Stability |
|---|---|---|---|---|---|---|
| 1 | Fluxible | - | 7.69 | ⊚ | ○ | - |
| 2 | Sticky | - | 7.67 | ⊚ | ○ | - |
| 3 | Partially sticky | - | 8.12 | ⊚ | ○ | - |
| 4 | Slightly sticky | 0.67 | 7.55 | ⊚ | ○ | ⊚ |
| 5 | Not sticky | 0.90 | 7.62 | ⊚ | ○ | ⊚ |
| 6 | Not sticky | 1.27 | 7.51 | ⊚ | ○ | ⊚ |
| 7 | Not sticky | 1.62 | 6.67 | ⊚ | ○ | - |

| Example (II) | Sense of touch | Hardness | pH value | Thermo-resistance | Cryo-resistance | Stability |
|---|---|---|---|---|---|---|
| 8 | Solution | - | 11.43 | ○ | ○ | - |
| 9 | Sticky | - | 11.72 | ○ | ○ | - |
| 10 | Not sticky | - | 11.71 | ○ | ○ | - |

| Example (III) | Sense of touch | Hardness | pH value | Thermo-resistance | Cryo-resistance | Stability |
|---|---|---|---|---|---|---|
| 11 | Slightly sticky | - | 6.60 | ⊚ | ⊚ | - |
| 12 | Not sticky | - | 8.12 | ⊚ | ⊚ | - |

| Example (IV) | Sense of touch | Hardness | pH value | Thermo-resistance | Cryo-resistance | Stability |
|---|---|---|---|---|---|---|
| 13 | Sticky | - | - | △ | × | - |
| 14 | Sticky | - | 6.17 | △ | × | - |
| 15 | Not sticky | - | 6.24 | ○ | × | - |

| Example (V) | Sense of touch | Hardness | pH value | Thermo-resistance | Cryo-resistance | Stability |
|---|---|---|---|---|---|---|
| 16 | Slightly sticky | - | - | ⊚ | ⊚ | ⊚ |

| Example (VI) | Sense of touch | Hardness 2K/20N | pH value | Thermo-resistance | Cryo-resistance | Stability |
|---|---|---|---|---|---|---|
| 17 | Sticky | 0.18 | 6.05 | ○ | ○ | - |
| 18 | Sticky | 0.15 | 7.68 | ⊚ | ○ | - |
| 19 | Sticky | 0.14 | - | ⊚ | ⊚ | ⊚ |
| 20 | Sticky | 0.11 | - | ○ | ⊚ | ⊚ |
| 21 | Sticky | 0.20 | - | ⊚ | ⊚ | ⊚ |
| 22 | Sticky | 0.29 | - | ⊚ | ⊚ | ⊚ |
| 23 | Sticky | 0.46 | - | ⊚ | ⊚ | - |

| | | | | | | |
|---|---|---|---|---|---|---|
| -: not available ⊚: The gel was very stable and could be normally used after being returned to room temperature with a high recovery speed ∘: The gel was stable and could be normally used after being returned to room temperature with a relatively high recovery speed △: the gel was not quite stable and the appearance changed after being returned to room temperature with compromised performance ×: the gel was unstable and could not be used normally after being returned to room temperature | | | | | | |

In summary, the combinations of N-long-chain acyl amino acids and arginine exhibited very good stability, wherein the combination of the N-lauroyl-L-alanine and the arginine exhibited good thermo-resistance, cryo-resistance and stability, and excellent cleansing capability; the combination of lauroyl sarcosine and arginine exhibited excellent thermo-resistance, cryo-resistance and stability, but compromised detergency.

The combination of N-lauroyl-L-alanine and lysine exhibited poor stability (particularly, cryo-stability); the combination of N-lauroyl-L-alanine and sodium hydroxide exhibited moderate stability.

The combination of N-lauroyl-L-alanine, lauroyl alanyl alanine (dipeptide), and arginine performed best. On one hand, the products exhibited excellent thermo-resistance, cryo-resistance, and stability; on the other hand, the product showed good detergency and desirability for use in various scenes.

Further studies have found that when the degree of neutralization is 85% or greater and less than 100%, a very soft gel is formed, which is more suitable for filling tubular products. An insufficient degree of neutralization (e.g., 85% or less) may sometimes lead to whitening in thermo- and cryo-resistance cycle tests (possibly, excessive unneutralized lauroyl alanine may be gradually precipitated). When a small amount of dipeptide is added and no neutralizing agent is added (Example 20), the use of about 25% of long-chain acyl amino acid may help form a stable soft gel.

Moreover, those skilled in the art will appreciate that although some embodiments described herein include some features included in other embodiments, the combinations of features in different embodiments shall fall within the scope of the present disclosure and form different embodiments. For example, in the Claims, any of the claimed embodiments may be used in arbitrary combinations.

## Claims

1. A gel composition, comprising: (a) a N-long-chain acyl amino acid, (b) a basic amino acid, and (c) water, wherein the gel is formed by self-thickening of the N-long-chain acyl amino acid and the basic amino acid;
or, comprising: (a) a N-long-chain acyl amino acid, (b) a basic amino acid, (c) water, and (d) a N-long-chain acyl amino acid dipeptide, wherein the gel is formed by self-thickening of the N-long-chain acyl amino acid, the basic amino acid, and the N-long-chain acyl amino acid dipeptide;
or, comprising: (a) a N-long-chain acyl amino acid, (e) an inorganic base, and (c) water, wherein the gel is formed by self-thickening of the N-long-chain acyl amino acid and the inorganic base;
or, comprising: (a) a N-long-chain acyl amino acid, (e) an inorganic base, (c) water, and (d) a N-long-chain acyl amino acid dipeptide, wherein the gel is formed by self-thickening of the N-long-chain acyl amino acid, the inorganic base, and the N-long-chain acyl amino acid dipeptide.

2. A gel composition, comprising: (a) a N-long-chain acyl amino acid, (b) a basic amino acid, and (c) water, wherein the percentage by weight of the N-long-chain acyl amino acid is 30 wt% or greater, preferably 33 wt% or greater, and more preferably 35 wt% or greater, and the percentage by weight of the basic amino acid is 15 wt% or greater, preferably 17 wt% or greater, and more preferably 20 wt% or greater;
or, comprising: (a) a N-long-chain acyl amino acid, (b) a basic amino acid, (c) water, and (d) a N-long-chain acyl amino acid dipeptide, wherein the sum of the percentages by weight of the N-long-chain acyl amino acid and N-long-chain acyl amino acid dipeptide is 20 wt% or greater, preferably 23 wt% or greater, and more preferably 25 wt% or greater, and the percentage by weight of the basic amino acid is 10 wt% or greater, preferably 12 wt% or greater, and more preferably 14 wt% or greater;
or, comprising: (a) a N-long-chain acyl amino acid, (b) a basic amino acid, and (c) water, wherein the percentage by weight of the N-long-chain acyl amino acid is 20 wt% or greater, preferably 23 wt% or greater, and more preferably 25 wt% or greater, and the molar ratio of the N-long-chain acyl amino acid to the basic amino acid is 1:0.98 to 1:0.85, preferably 1:0.96 to 1:0.88, and more preferably 1:0.95 to 1:0.90.

3. A gel composition, comprising: (a) a N-long-chain acyl amino acid, (e) an inorganic base, and (c) water, wherein the percentage by weight of the N-long-chain acyl amino acid is 40 wt% or greater, preferably 43 wt% or greater, and more preferably 45 wt% or greater, and the percentage by weight of the inorganic base is 5.5 wt% or greater, preferably 6 wt% or greater, and more preferably 6.5 wt% or greater;
or, comprising: (a) a N-long-chain acyl amino acid, (e) an inorganic base, (c) water, and (d) a N-long-chain acyl amino acid dipeptide, wherein the sum of the percentages by weight of the N-long-chain acyl amino acid and N-long-chain acyl amino acid dipeptide is 30 wt% or greater, preferably 33 wt% or greater, and more preferably 35 wt% or greater, and the percentage by weight of the inorganic base is 4 wt% or greater, preferably 4.5 wt% or greater, and more preferably 5 wt% or greater;
or, comprising: (a) a N-long-chain acyl amino acid, (e) an inorganic base, and (c) water, wherein the percentage by weight of the N-long-chain acyl amino acid is 30 wt% or greater, preferably 33 wt% or greater, and more preferably 35 wt% or greater, and the molar ratio of the N-long-chain acyl amino acid to the inorganic base is 1:0.98 to 1:0.85, preferably 1:0.96 to 1:0.88, and more preferably 1:0.95 to 1:0.90.

4. The gel composition according to any one of claims 1-3, wherein the N-long-chain acyl group in the N-long-chain acyl amino acid is derived from a saturated or unsaturated linear or branched fatty acid with 8-22 carbon atoms;
and/or, the amino acid in the N-long-chain acyl amino acid is derived from one or more of glycine, alanine, glutamic acid, sarcosine, aspartic acid, leucine, isoleucine, valine, threonine, proline, phenylalanine, arginine, lysine, or (methyl) taurine.

5. The gel composition according to any one of claims 1-4, wherein the N-long-chain acyl group in the N-long-chain acyl amino acid is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl;
and/or, the amino acid in the N-long-chain acyl amino acid is derived from one or more of alanine, glycine, glutamic acid, sarcosine, arginine, lysine, or (methyl) taurine, preferably alanine, and most preferably L-alanine.

6. The gel composition according to any one of claims 1-5, wherein the basic amino acid is selected from one or more of arginine, lysine, citrulline, ornithine, creatine, histidine, diaminobutyric acid, and diaminopropionic acid, preferably arginine and/or lysine, and most preferably arginine;
and/or, the inorganic base is selected from one or more of sodium hydroxide and potassium hydroxide.

7. The gel composition according to any one of claims 1-6, wherein the N-long-chain acyl group in the N-long-chain acyl amino acid dipeptide is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, or palm oil fatty acyl, preferably coconut oil fatty acyl or lauroyl, and most preferably lauroyl;
and/or, the amino acids in the N-long-chain acyl amino acid dipeptide are derived from one or more of alanine, glycine, glutamic acid, sarcosine, arginine, lysine, or (methyl) taurine, preferably alanine, and most preferably L-alanine.

8. The gel composition according to any one of claims 1-7, wherein the N-long-chain acyl amino acid dipeptide is selected from one or more of long-chain acyl glycyl glycine, long-chain acyl glutamyl glutamic acid, long-chain acyl alanyl alanine, and long-chain acyl sarcosyl sarcosine.

9. The gel composition according to any one of claims 2 and 4-8, wherein the percentage by weight of the N-long-chain acyl amino acid is 55 wt% or less, preferably 50 wt% or less, and more preferably 45 wt% or less.

10. The gel composition according to any one of claims 3-8, wherein the percentage by weight of the N-long-chain acyl amino acid is 75 wt% or less, preferably 65 wt% or less, and more preferably 60 wt% or less.

11. The gel composition according to any one of claims 1-10, wherein the percentage by weight of the N-long-chain acyl amino acid dipeptide in the composition is 0.5 wt% or greater, preferably 0.75 wt% or greater, and more preferably 1 wt% or greater;
and/or, the mass ratio of the N-long-chain acyl amino acid to the N-long-chain acyl amino acid dipeptide is 20:1 to 1.5:1, preferably 15:1 to 3:1, and more preferably 10:1 to 6:1.

12. The gel composition according to any one of claims 1-11, wherein the basic amino acid or the inorganic base is present in an amount sufficient to partially or completely neutralize the N-long-chain acyl amino acid and N-long-chain acyl amino acid dipeptide, preferably with a degree of neutralization of 85% or greater and less than 100%, and more preferably with a degree of neutralization of 88% or greater and less than 100%.

13. The gel composition according to any one of claims 1-12, wherein the composition is free or substantially free of a thickening agent;
and/or, the composition is free or substantially free of sodium chloride.

14. The gel composition according to claim 13, wherein the thickening agent is selected from one or more of cellulose, a mucopolysaccharide, polyvinyl alcohol, a gum, sodium polyacrylate, polyvinyl pyrrolidone, and a vinyl polymer.

15. The gel composition according to any one of claims 1-14, wherein the gel composition has a pH value of 4.5 or greater, preferably 5 or greater, and more preferably 5.5 or greater, and 13 or less, preferably 11 or less, and more preferably 10 or less, as measured by the dilution method specified in GB/T 13531.1.

16. The gel composition according to any one of claims 1-15, wherein the composition, after forming a gel, has a hardness of 1.80 or less, preferably 1.50 or less, and more preferably 1.00 or less, as measured by RHEOTECH (Japan) durometer in the conditions of a load of 2 kg, an adapter of 20 mmφ, a penetration speed of 6 cm/min, and a penetration distance of 10 mm.

17. The gel composition according to any one of claims 1-16, wherein the composition further comprises a water-soluble grease and/or an alkyl polyglycoside.

18. The gel composition according to claim 17, wherein the water-soluble grease is selected from one or more of a PEG olive oil ester, a PEG isostearate, a PEG cocoyl ester, and a PEG avocado oil ester; preferably one or more of a PEG-10 olive oil glyceride, an olive oil PEG-7 ester, an olive oil PEG-8 ester, a PEG-7 olive oil ester, a PEG-15 glyceryl isostearate, a PEG-9 cocoglyceride, and a PEG-11 avocado glyceride.

19. A gel, wherein the gel is formed on the basis of the self-thickening of a N-long-chain acyl amino acid and a basic amino acid;
or, on the basis of the self-thickening of a N-long-chain acyl amino acid, a basic amino acid, and a N-long-chain acyl amino acid dipeptide;
or, on the basis of the self-thickening of a N-long-chain acyl amino acid and an inorganic base;
or, on the basis of the self-thickening of a N-long-chain acyl amino acid, an inorganic base, and a N-long-chain acyl amino acid dipeptide.

20. A gel, wherein the gel is prepared from the gel composition according to any one of claims 1-18.

21. The gel according to claim 19 or 20, wherein the gel can be used normally after being kept at 40 ± 1 °C for 24 hours and returned to room temperature;
and/or, the gel can be used normally after being kept at -8 ± 2 °C for 24 hours and returned to room temperature.

22. The gel according to any one of claims 19-21, wherein the gel is a cleansing gel, a skin care gel, a shampoo gel, a body wash gel, a shaving gel, a makeup remover gel, a massage gel, a make-up gel, a cosmetic gel, or a multifunctional gel.

23. Use of the gel composition according to any one of claims 1-18 in a cleansing composition, a detergent composition, a cosmetic composition, a skin care composition, or an oral care composition.

24. A toothpaste, comprising the gel composition according to any one of claims 1-18.

25. A cosmetic product, comprising the gel composition according to any one of claims 1-18.

26. A cleansing product, comprising the gel composition according to any one of claims 1-18.

27. A method for preparing the gel composition according to any one of claims 1-18, comprising:
mixing (a) a N-long-chain acyl amino acid, (b) a basic amino acid or (e) an inorganic base, (c) water, and optionally (d) a N-long-chain acyl amino acid dipeptide at 75-90 °C, preferably 80-85 °C,
wherein preferably, components (b) or (e) and (c) are first mixed to give solution I, then (a) and optionally (d) are melted at 75-90 °C, preferably 80-85 °C, to give solution II, and finally the solution I and solution II are mixed at 75-90 °C, preferably 80-85 °C.

28. A method for imparting gel characteristics to a cleansing composition, wherein the cleansing composition comprises (a) a N-long-chain acyl amino acid and (b) a basic amino acid; the percentage by weight of the N-long-chain acyl amino acid is 30 wt% or greater, preferably 33 wt% or greater, and more preferably 35 wt% or greater, and the percentage by weight of the basic amino acid is 15 wt% or greater, preferably 17 wt% or greater, and more preferably 20 wt% or greater;
or, the cleansing composition comprises (a) a N-long-chain acyl amino acid, (b) a basic amino acid, and (d) a N-long-chain acyl amino acid dipeptide; the sum of the percentages by weight of the N-long-chain acyl amino acid and N-long-chain acyl amino acid dipeptide is 20 wt% or greater, preferably 23 wt% or greater, and more preferably 25 wt% or greater, and the percentage by weight of the basic amino acid is 10 wt% or greater, preferably 12 wt% or greater, and more preferably 14 wt% or greater;
or, the cleansing composition comprises (a) a N-long-chain acyl amino acid and (b) a basic amino acid; the percentage by weight of the N-long-chain acyl amino acid is 20 wt% or greater, preferably 23 wt% or greater, and more preferably 25 wt% or greater, and the molar ratio of the N-long-chain acyl amino acid to the basic amino acid is 1:0.98 to 1:0.85, preferably 1:0.96 to 1:0.88, and more preferably 1:0.95 to 1:0.90.

29. A method for imparting gel characteristics to a cleansing composition, wherein the cleansing composition comprises (a) a N-long-chain acyl amino acid and (e) an inorganic base; the percentage by weight of the N-long-chain acyl amino acid is 40 wt% or greater, preferably 43 wt% or greater, and more preferably 45 wt% or greater, and the percentage by weight of the inorganic base is 5.5 wt% or greater, preferably 6 wt% or greater, and more preferably 6.5 wt% or greater;
or, the cleansing composition comprises (a) a N-long-chain acyl amino acid, (e) an inorganic base, and (d) a N-long-chain acyl amino acid dipeptide; the sum of the percentages by weight of the N-long-chain acyl amino acid and N-long-chain acyl amino acid dipeptide is 30 wt% or greater, preferably 33 wt% or greater, and more preferably 35 wt% or greater, and the percentage by weight of the inorganic base is 4 wt% or greater, preferably 4.5 wt% or greater, and more preferably 5 wt% or greater;
or, the cleansing composition comprises (a) a N-long-chain acyl amino acid and (e) an inorganic base; the percentage by weight of the N-long-chain acyl amino acid is 30 wt% or greater, preferably 33 wt% or greater, and more preferably 35 wt% or greater, and the molar ratio of the N-long-chain acyl amino acid to the inorganic base is 1:0.98 to 1:0.85, preferably 1:0.96 to 1:0.88, and more preferably 1:0.95 to 1:0.90.
